(19) Europäisches Patentamt
European Patent Office
Office européen des brevets

(11) EP 1 272 472 B1

(12) **EUROPÄISCHE PATENTSCHRIFT**

(45) Veröffentlichungstag und Bekanntmachung des Hinweises auf die Patenterteilung:
**16.03.2005 Patentblatt 2005/11**

(21) Anmeldenummer: **01919446.3**

(22) Anmeldetag: **10.04.2001**

(51) Int Cl.[7]: **C07D 231/12**, C07D 231/16, C05G 3/08, C07D 521/00

(86) Internationale Anmeldenummer:
**PCT/EP2001/004117**

(87) Internationale Veröffentlichungsnummer:
**WO 2001/079178 (25.10.2001 Gazette 2001/43)**

(54) **1H-PYRAZOL-DERIVATE, VERFAHREN ZU IHRER HERSTELLUNG UND DEREN VERWENDUNG ZUR HEMMUNG UND STEUERUNG DER NITRIFIKATION**

1H-PYRAZOLE DERIVATIVES, METHOD FOR THE PRODUCTION THEREOF AND THEIR USE FOR INHIBITING AND CONTROLLING NITRIFICATION

DERIVES DE 1H-PYRAZOL, PROCEDES PERMETTANT DE LES PREPARER ET LEUR UTILISATION POUR INHIBER ET REGULER LA NITRIFICATION

(84) Benannte Vertragsstaaten:
**AT BE CH CY DE DK ES FI FR GB GR IE IT LI LU MC NL PT SE TR**

(30) Priorität: **14.04.2000 DE 10018604**

(43) Veröffentlichungstag der Anmeldung:
**08.01.2003 Patentblatt 2003/02**

(73) Patentinhaber: **SKW STICKSTOFFWERKE PIESTERITZ GmbH**
**06886 Lutherstadt Wittenberg (DE)**

(72) Erfinder:
- **RADICS, Ute**
  **06888 Dabrun (DE)**
- **NICLAS, Hans-Joachim**
  **12435 Berlin (DE)**
- **MICHEL, Hans-Jürgen**
  **04827 Machern (DE)**
- **KRISTOF, Wolfgang**
  **06886 Lutherstadt Wittenberg (DE)**
- **GRABARSE, Margit**
  **04687 Seelingstädt (DE)**

(74) Vertreter: **Schneider, Michael**
**HAMMONDS,**
**Karl-Scharnagl-Ring 7**
**80539 München (DE)**

(56) Entgegenhaltungen:
**EP-A- 0 278 445**
**WO-A-98/05607**
**DE-A- 19 822 340**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 272 472 B1

Printed by Jouve, 75001 PARIS (FR)

## Beschreibung

Die vorliegende Erfindung betrifft die Verwendung von 1H-Pyrazol-Derivaten zur Hemmung bzw. Steuerung der Nitrifikation, Düngemittelzusammensetzungen die diese enthalten, neue 1H-1-(Cyclo-)Alkoxymethyl-pyrazole sowie Verfahren zu deren Herstellung.

Die Anwendung von Stickstoffdüngemitteln, die Nitrifikationsinhibitoren enthalten, ist dadurch besonders vorteilhaft, dass die im allgemeinen rasch ablaufende Nitrifikation, d. h. die mikrobielle Umwandlung von Amid- und Ammoniumstickstoff zu Nitratstickstoff, auf diese Weise verzögert wird. Die derart gesteuerte Nitrifikation im Boden vermeidet eine zu intensive Nitratbildung und damit die Akkumulation von Nitrat. Eine so bewirkte Zurückdrängung des sonst entstehenden Nitratüberschusses im Boden konserviert den Düngerstickstoff in Form von Ammoniumionen, sorgt damit für eine höhere Düngereffizienz und führt gleichzeitig zu einer erheblich verringerten Umweltbelastung im Rahmen einer Stickstoffdüngung.

So ist bekannt, dass Nitrat im Gegensatz zum Ammonium im Boden nur äußerst schlecht sorbiert wird und demzufolge der Auswaschung bzw. der Verlagerung in tiefere Bodenschichten unterliegt. Andererseits führt die infolge einer Nitratanreicherung im Boden verstärkt einsetzende Denitrifikation zu gasförmigen Stickstoffverlusten, wobei neben molekularem Stickstoff auch solche Oxide wie Stickstoffmonoxid bzw. Lachgas emittiert werden, die zur Versauerung des Bodens beitragen bzw. als klimaveränderndes Gas bekannt sind. Gleichzeitig wird einer Nitratanreicherung im Grundwasser entgegengewirkt.

Gerade in der Reduzierung der beschriebenen Verlustpotentiale liegt der wirtschaftliche Vorteil von mit Nitrifikationsinhibitoren versehenen Stickstoffdüngemitteln, der sich in der erhöhten Nährstoffeffizienz dieser Dünger niederschlägt. Um das zu unterstreichen, sei erwähnt, dass N-Verluste von konventionellen Stickstoffdüngern durchaus unter ungünstigen Bedingungen Größenordnungen von bis zu 40 % und mehr annehmen können.

Als wirksame Nitrifikationshemmer wurden unter anderem stickstoffhaltige heterocyclische Verbindungen, bevorzugt 1H-Pyrazole und 1H-1,2,4-Triazole, vorgeschlagen (US 3,635,690, US 4,969,946, US 4,523,940, EP-A 166 420, JP-OS 71-04135, US 3,697,244) . DE-A-198 22 340 beschreibt Pyrazolderivate mit Amingruppen-haltigen substituenten.

Ein wesentlicher Nachteil der meisten sehr gut nitrifikationshemmend wirkenden Pyrazolderivate besteht in ihrer relativ hohen Flüchtigkeit bzw. besonders bei an N-1-Stickstoffatom substituierten Verbindungen in ihrer Hydrolyseempfindlichkeit, in deren Folge zwar noch wirksame, aber wieder flüchtige Derivate gebildet werden. Aus diesen Gründen lässt sich eine Anwendung bei festen Düngemitteln, insbesondere in Kombinationen mit Harnstoff, nicht ohne weiteres realisieren. Zur Beseitigung dieses Mangels sind Vorschläge zur chemischen Abwandlung der 1-N-unsubstituierten Basispyrazole bekannt (DE-OS 27 45 833, EP-A 508 191, DD292 232, DE OS 40 18 395, EP-A 160 804, DE-OS 37 04 359, SU 1 470 732, DE-OS 44 46 194, EP-A 808 297). Mit dem Rückgang der Flüchtigkeit durch Derivatisierung ist jedoch in der Regel zugleich auch ein Abfall in der Wirkung als Nitrifikationsinhibitor verbunden.

Andere Lösungen zur Reduzierung der Flüchtigkeit von nitrifikationshemmend wirkenden Pyrazolen bestehen in der Ausnutzung des eigentlich bekannten Prinzips der Salzbildung mit Säuren (EP-A 529 473, WO 98/05 607, DE-OS 4018 395).

Sowohl die mineralsauren Salze der nitrifiziden Basispyrazole als auch nitrifizid wirkende Pyrazole, die am 1-N-Stickstoff hydrophile Gruppen tragen, können als Nitrifikationsinhibitoren noch nicht voll befriedigen, da sie eine zu geringe Hydrolysestabilität besitzen, wodurch die Wirkungsdauer im Boden und die Lagerstabilität herabgesetzt ist. Dies betrifft z. B. N-Glyoxylpyrazole (DE-A 37 04 359) oder N-Hydroxymethyl-pyrazole (SU-A 1 470 737).

Der vorliegenden Erfindung lag die Aufgabe zugrunde, Wirkstoffe zur Hemmung bzw. Steuerung der Nitrifikation von Ammoniumstickstoff zu entwickeln, die eine ausreichende Residualwirkung aufweisen und die aufgrund ihrer geringen Flüchtigkeit, ihrer Thermo- und Hydrolysestabilität alle Applikationsverfahren, aber auch alle Möglichkeiten zur Einarbeitung in oder Aufbringung auf mineralische Stickstoffhaltige Dünger zulassen.

Diese Aufgabe wurde erfindungsgemäß dadurch gelöst, dass man 1H-Pyrazol-Derivate sowie ihre Säureadditionsprodukte entsprechend Anspruch 1 verwendet.

Es hat sich nämlich überraschenderweise gezeigt, dass diese Verbindungen neben der guten nitrifikationshemmenden Wirkung den außerordentlichen Vorteil einer sehr geringen Flüchtigkeit besitzen, wodurch Wirkstoffverluste bei der Oberflächenapplikation aber auch bei der Lagerung von Dünger-Wirkstoff-Gemischen nahezu ausgeschlossen sind.

Die erfindungsgemäß verwendeten 1H-Pyrazol-Derivate entsprechen der allgemeinen Formel 1

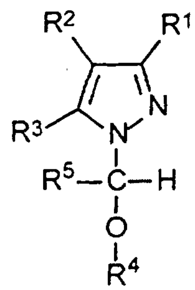

(I)

wobei $R^1$ und $R^3$ unabhängig voneinander für H oder einen $C_1$-$C_4$-Alkylrest stehen, und $R^2$ für H, Halogen (F, Cl, Br, I) oder einen $C_1$-$C_4$-Alkylrest steht.

Die $C_1$- bis $C_4$- Alkylreste für $R^1$, $R^2$ und $R^3$ können linear oder verzweigt sein. $R^1$ und $R^2$ können auch einen fünf- oder sechsgliedrigen Cycloalkylrest ausbilden.

Vorzugsweise stehen $R^1$ für einen $C_1$-$C_4$-Alkylrest,
$R^2$ für H, Halogen oder einen $C_1$-$C_4$-Alkylrest,
oder $R^1$ und $R^2$ zusammen für einen fünf- oder sechsgliedrigen Cycloalkylrest, und $R^3$ für H.

Besonders bevorzugt stellen $R^1$ und $R^2$ einen Methylrest dar.

Für $R^4$ und $R^5$ kommen als $C_1$-$C_{20}$-Alkylreste lineare oder verzweigte Alkylreste in Frage. $R^4$ und $R^5$ können auch für einen $C_3$-$C_8$-Cycloalkyl-, $C_6$-$C_{10}$-Aryl- oder Alkylarylrest mit $C_1$-$C_4$-Alkyl- und $C_6$-$C_{10}$-Arylgruppen stehen, wobei die Alkyloder Arylreste durch Halogen (F, Cl, Br, I), Hydroxyl, durch eine Trimethylsilyl-, Amino-, Nitro-, Cyano-, Carbonyl-, Carboxyl- oder eine Carboxyalkyl-gruppe mit 1 bis 5 C-Atomen substituiert sein können. $R^5$ kann außerdem noch für H stehen.

Bevorzugt steht $R^4$ für $C_1$-$C_8$-Alkyl, $C_5$-$C_6$-Cycloalkyl oder $C_6$-Aryl, und $R^5$ steht für H, $C_1$-$C_8$-Alkyl, $C_5$-$C_6$-Cycloalkyl oder $C_6$-Aryl.

Anstelle der 1H-Pyrazol-Derivate können auch deren Säureadditionsprodukte, insbesondere in Form der Umsetzungsprodukte mit Salzsäure, Phosphorsäure oder Schwefelsäure, verwendet werden.

Die erfindungsgemäß vorgeschlagenen Verbindungen sind in bekannter Weise als Nitrifikationsinhibitoren mit festen oder flüssigen ammonium- und/oder amidhaltigen Düngemitteln kombinierbar. Solche Zusammensetzungen bilden einen weiteren Aspekt der vorliegenden Erfindung. Als anorganische Stickstoffdüngemittel werden vorzugsweise Ammoniumsalze, wie z. B. Kalkammonsalpeter, Ammoniumsulfatsalpeter, Ammoniumsulfat, Ammoniumnitrat oder Harnstoff, eingesetzt.

Möglich ist auch der Einsatz der erfindungsgemäß vorgeschlagenen Verbindungen in Kombination mit organischen Düngemitteln. Aufwandmengen von 0,1 bis 5 Gew.-%, insbesondere 0,5 bis 3,0 Gew.-%, berechnet auf den reduzierten Stickstoffgehalt des Düngers, d. h. auf Ammoniumund Carbamidstickstoff, genügen für den praktischen Einsatz.

Die Art und Weise, wie die erfindungsgemäß vorgeschlagenen Nitrifikationsinhibitoren mit dem Düngemittel kombiniert werden, ist relativ unkritisch. So können die 1H-Pyrazol-Derivate bspw. in fester oder flüssiger Form, allein oder im Gemisch mit weiteren Zusatzstoffen in die Düngemittelschmelze oder -slurry während des Formgebungsprozesses eingebracht werden, um auf diese Weise homogen im Dünger verteilt vorzuliegen.

Alternativ hierzu können die 1H-Pyrazol-Derivate oder ihre Säureadditionsprodukte als Lösung auf die Oberfläche der Düngemittelgranulate aufgebracht werden, wobei das Lösemittel (vorzugsweise aprotische Lösemittel, wie z. B. Ketone, aliphatische Kohlenwasserstoffe, Ether etc.) durch Trocknung entfernt wird. Schließlich ist es im Rahmen der vorliegenden Erfindung auch möglich, die 1H-Pyrazol-Derivate oder deren Säureadditionsprodukte direkt dem Flüssigdünger zuzusetzen.

Ein weiterer Gegenstand der vorliegenden Erfindung sind 1H-Pyrazol-Derivate der allgemeinen Formel I

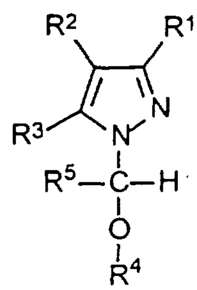

(I)

sowie deren Säureadditionsprodukte, wobei $R^1$ für einen $C_1$-$C_4$-Alkylrest, $R^2$ für H, Halogen (F, Cl, Br, I) oder einen $C_1$-$C_4$-Alkylrest, $R^1$ und $R^2$ zusammen für einen fünf- oder sechsgliedrigen Cycloalkylrest, $R^3$ für H, $R^4$ für $C_1$-$C_{20}$-Alkyl, $C_3$-$C_8$-Cycloalkyl, $C_6$-$C_{10}$-Aryl oder Alkylaryl mit $C_1$-$C_4$-Alkylund $C_6$-$C_{10}$-Arylgruppen und $R^5$ für H, $C_1$-$C_{20}$-Alkyl, $C_3$-$C_8$-Cycloalkyl, $C_6$-$C_{10}$-Aryl oder Alkylaryl mit $C_1$-$C_4$-Alkyl- und $C_6$-$C_{10}$-Arylgruppen stehen.

Die $C_1$- bis $C_4$ -Alkylreste für $R^1$ und $R^2$ können linear oder verzweigt sein. Vorzugsweise stellen $R^1$ und $R^2$ einen Methylrest dar. Als $C_1$-$C_{20}$-Alkylreste kommen für $R^4$ und $R^5$ lineare oder verzweigte Alkylreste in Frage. $R^4$ und $R^5$ können auch für einen $C_3$-$C_8$-Cycloalkyl-, $C_6$-$C_{10}$-Aryl- oder Alkylarylrest mit $C_1$-$C_4$-Alkyl- und $C_6$-$C_{10}$-Arylgruppen stehen, wobei die Alkyl- oder Arylreste durch Halogen (F, Cl, Br, I), Hydroxyl, durch eine Trimethylsilyl-, Amino-, Nitro-, Cyano-, Carbonyl-, Carboxyl- oder eine Carboxyalkyl-gruppe mit 1 bis 5 C-Atomen substituiert sein können.

Bevorzugt steht $R^4$ für $C_1$-$C_8$-Alkyl, $C_5$-$C_6$-Cycloalkyl oder $C_6$-Aryl, und $R^5$ steht für H, $C_1$-$C_8$-Alkyl, $C_5$-$C_6$-Cycloalkyl oder $C_6$-Aryl.

Die erfindungsgemäß vorgeschlagenen 1H-Pyrazol-Derivate (I) sind aus dem 1-unsubstituierten Basispyrazol (II) (wobei $R^1$, $R^2$ und $R^3$ oben genannte Bedeutung besitzen) durch Reaktion mit einem α-Chlorether (III) (wobei $R^4$ und $R^5$ oben genannte Bedeutung besitzen) gemäß der allgemeinen Gleichung (1) zugänglich, wobei man das entsprechende Pyrazol mit dem α-Chlorether im Molverhältnis 1 : 1,0 bis 1,2 unter Zusatz von vorzugsweise molaren Mengen an Basen, wie z. B. Alkalicarbonat (insbesondere Natrium- und Kaliumcarbonat) in einem organischen Lösemittel ausgewählt aus der Gruppe Ketone (wie z. B. Aceton) oder aromatische Kohlenwasserstoffe (vorzugsweise Toluol) bei Temperaturen von 40 bis 100 °C umsetzt.

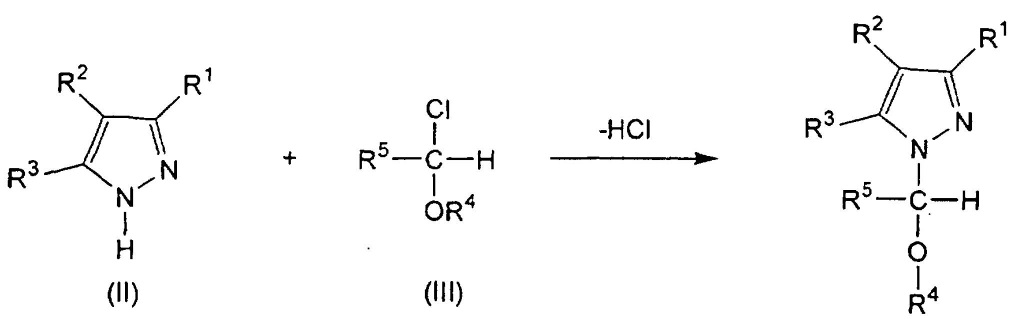


(II) (III) (I) (1)

Die 1H-Pyrazol-Derivate können auch erhalten werden, indem man

a) 1-unsubstituierte Basispyrazole (II) (wobei $R^1$, $R^2$ und $R^3$ oben genannte Bedeutung besitzen) zunächst mit Aldehyden der allgemeinen Formel (IV) (wobei $R^5$ die oben genannte Bedeutung besitzt) gemäß der allgemeinen Gleichung (2) umsetzt, wobei das entsprechende Pyrazol mit dem Aldehyd im Molverhältnis 1 : 1,0 bis 1,2 gegebenenfalls in Gegenwart eines organischen Lösemittels (vorzugsweise ausgewählt aus der Gruppe Halogenkohlenwasserstoffe, wie z.B. Methylenchlorid, Alkohole, wie z.B. Ethanol, oder aromatische Kohlenwasserstoffe, bevorzugt Toluol) bei Temperaturen von -30 bis 120 °C zur Reaktion gebracht wird und

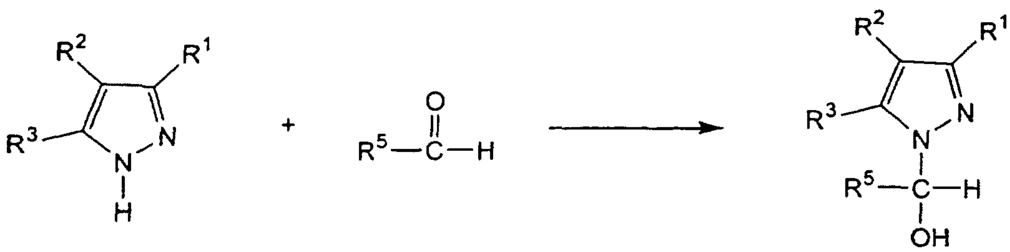

(II) (IV) (V) (2)

b) die dabei entstehenden Pyrazol-Derivate der allgemeinen Formel (V), wobei $R^1$, $R^2$, $R^3$ und $R^5$ oben genannte Bedeutung besitzen, entweder direkt (wobei der Alkohol $R^4$OH dem Reaktionsgemisch vorzugsweise bei Temperaturen von -30 bis 10 °C zugegeben wird) oder gegebenenfalls nach Isolierung der Produkte (V) mit dem entsprechenden Alkohol $R^4OH$ unter Anwendung von dem Fachmann bekannten Etherbildungsmethoden (Methoden der Organischen Chemie, Houben-Weyl, 4.Auflage 1965, Band 6/3) gemäß der allgemeinen Gleichung (3) verethert werden. Dabei wird das jeweiligen Pyrazol-Derivat (V) mit dem entsprechenden Alkohol $R^4OH$ (wobei $R^4$ für $C_1$-$C_{20}$-Alkyl, $C_3$-$C_8$-Cycloalkyl steht) im bevorzugten Molverhältnis 1 : 1,0 bis 1,2 unter Zusatz von sauren Katalysatoren in aromatischen Kohlenwasserstoffen (wie z.B. Toluol) als Lösungsmittel bei Temperaturen von 80 bis 150 °C umgesetzt. Als besonders günstig haben sich als Katalysator p-Toluolsulfonsäure und saure Ionenaustauscher erwiesen.

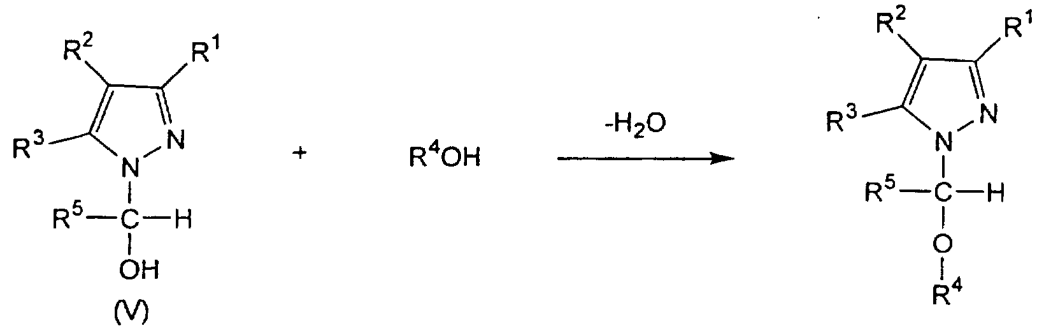


(V) (I) (3)

Außerdem können die erfindungsgemäßen Pyrazol-Derivate (I) ($R^5 \neq H$) auch durch Erhitzen der 1-unsubstituierten Pyrazol-Derivate (II) (wobei $R^1$, $R^2$ und $R^3$ oben genannte Bedeutung besitzen) mit überschüssigem Aldehyddiacetal (VI) (wobei $R^4$ und $R^5$ oben genannte Bedeutung besitzen und $R^5 \neq H$ ist) im Molverhältnis 1 : 2 bis 5 unter Verwendung von sauren Katalysatoren, bevorzugt p-Toluolsulfonsäure, bei Temperaturen von 100 - 130 °C gemäß der allgemeinen Gleichung (4) hergestellt werden.

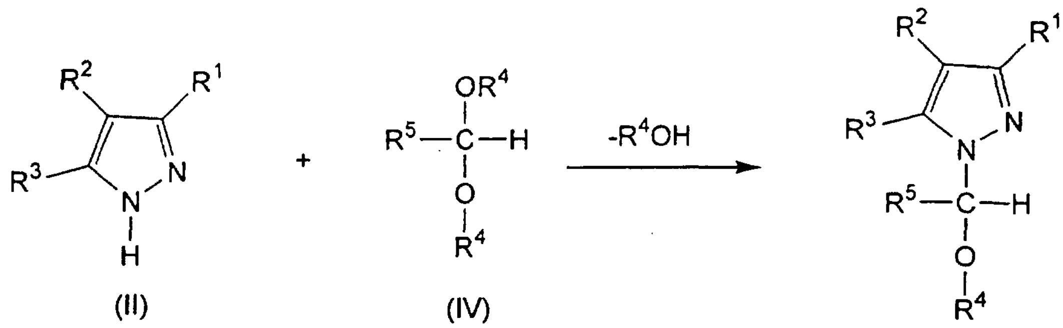


(II) (IV) (I) (4)

Die Umsetzung der Pyrazol-Derivate (V) (wobei $R^1$, $R^2$, $R^3$ und $R^5$ oben genannte Bedeutung besitzen) mit den entsprechenden Alkylhalogeniden $R^4Hal$ (wobei $R^4$ für $C_1$-$C_{20}$-Alkyl, $C_3$-$C_8$-Cycloalkyl steht) im Molverhältnis 1 : 1 bis 3, unter Verwendung von vorzugsweise äquimolaren Mengen Alkalicarbonat (z.B. Natrium- oder Kaliumcarbonat) gemäß der allgemeinen Gleichung (5) bei Temperaturen von 50 bis 130 °C ist eine weitere Synthesevariante für die erfindungsgemäßen Pyrazol-Derivate (I).

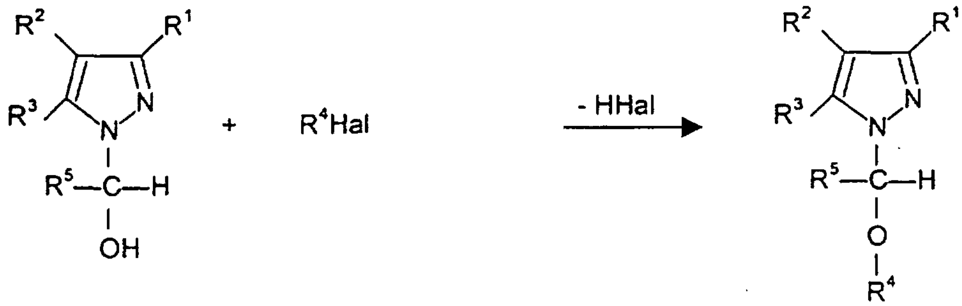


(V) (I) (5)

Die Reaktionsprodukte sind in den meisten Fällen destillierbare Flüssigkeiten, die mit zunehmender Molekülgröße viskoser werden.

Im Falle von Umsetzungen entsprechend Gleichung (1), in denen bei den Ausgangspyrazolen eine Tautomerie möglich ist, wie z. B. beim 3(5)-Methylpyrazol, beim 3(5),4-Dimethylpyrazol oder beim 4-Chlor-3(5)-methylpyrazol, wurden die bei der Synthese über NMR-Untersuchungen nachgewiesenen Isomeren nicht getrennt. Es wurden im Durchschnitt ca. 55 bis 60 % 3-Methyl- und 40 bis 45 % 5-Methyl-Derivate erhalten. Bei der Herstellung der erfindungsgemäßen Verbindungen unter Verwendung der Pyrazol-Derivate (V) als Ausgangsprodukte entsprechend Gleichung (3) und (5), wurde von vornherein von isomeren Mischungen ausgegangen.

Die erfindungsgemäßen Mittel zeichnen sich durch eine hohe thermische Belastbarkeit aus, die eine Inkorporation in das Düngemittel vor dessen Formgebung über den schmelzflüssigen Zustand unter Beibehaltung der guten nitrifikationshemmenden Wirkung ermöglicht. Die Einführung des hydrophoben Restes - $CHR^5OR^4$ führt zu einem erheblichen Anstieg der Hydrolysestabilität der Verbindungen, ohne dass ein Rückgang der nitrifiziden Wirkung erfolgt. Durch ihren hydrophoben Charakter sind die Wirkstoffe außerdem für eine alternative Aufbringung auf die Düngeroberfläche sehr gut geeignet.

Diese als äußerst vorteilhaft zu bewertenden Eigenschaften der erfindungsgemäßen Pyrazole werden auch durch ihre Überführung in die korrespondierenden Säureadditionsprodukte nicht beeinträchtigt. Vielmehr wird dadurch eine ausreichende Wasserlöslichkeit induziert, die ihre Anwendung auch für stickstoffhaltige Flüssigdünger ermöglicht.

Die nachfolgenden Beispiele sollen die Erfindung näher veranschaulichen.

Beispiele

Darstellung von 1H-Pyrazol-Derivaten

Variante A

Allgemeine Synthesevorschrift:

0,1 mol Pyrazol (II) werden in 185 ml Aceton gelöst vorgelegt und 0,1 mol wasserfreies Kaliumcarbonat zugegeben. Die Mischung wird auf 10 °C abgekühlt und bei dieser Temperatur 0,1 mol Chlormethylether zugetropft. Man lässt eine Stunde nachrühren und erhitzt anschließend 40 Minuten unter Rückfluss. Nach dem Abkühlen wird vom Feststoff abgesaugt und das Lösemittel unter Vakuum entfernt. Der Rückstand wird in 75 ml Wasser gelöst und dreimal mit Methylenchlorid extrahiert. Die vereinigten organischen Phasen werden mit gesättigter Natriumchloridlösung gewaschen, getrocknet und eingedampft. Der verbliebene Rückstand wird durch Destillation oder Säulenchromatographie gereinigt.

Variante B

Allgemeine Synthesevorschrift:

Eine Mischung aus 0,5 mol Pyrazol-Derivat (V), 0,5 mol Alkohol $R^4OH$, 0,2 mol% p-Toluolsulfonsäure und 40 ml Toluol wird solange unter Rückfluss erhitzt, bis sich kein Wasser mehr abscheidet. Die leichtflüchtigen Anteile werden durch eine Vakuumdestillation aus dem Reaktionsgemisch entfernt und der Rückstand durch Säulenchromatographie gereinigt.

Variante C

Allgemeine Synthesevorschrift

Zu 0,2 mol Pyrazol (II) in 100 ml Toluol werden bei -10 bis 0°C 0,22 mol Aldehyd (IV) in 20 ml Toluol dosiert. Man lässt eine Stunde bei dieser Temperatur nachrühren und gibt dann bei 0°C 0,22 mol Alkohol $R^4OH$ und 0,2 mol % p-Toluolsulfonsäure zu. Die Kühlung wird entfernt und nach einer halben Stunde wird solange unter Rückfluss erhitzt, bis sich kein Wasser mehr abscheidet. Die leichtflüchtigen Anteile werden durch eine Vakuumdestillation aus dem Reaktionsgemisch entfernt und der Rückstand durch Säulenchromatographie gereinigt.

Variante D

Allgemeine Synthesevorschrift

0,1 mol Pyrazol (II), 0,2 mol Aldehyddiacetal (VI) und 0,2 mol % p-Toluolsulfonsäure werden auf 100 - 130 °C erhitzt. Es wird langsam über eine Vigreux-Kolonne abdestilliert Der verbleibende Rückstand wird durch Säulenchromatographie gereinigt.

Die in Tabelle 1 aufgeführten Substanzen wurden nach obigen Synthesevarianten erhalten, wobei gleichzeitig unterstrichen wird, dass zum Erhalt der erfindungsgemäßen Verbindungen prinzipiell alle Varianten geeignet sind. Die Strukturen der aufgeführten Verbindungen wurden durch NMR-Spektroskopie und Elementaranalyse gesichert.

Tabelle 1: 1H-1-Alkoxymethyl-pyrazole

| Nr. | $R^1$ | $R^2$ | $R^3$ | $R^4$ | $R^5$ | Var. | Ausb. (%) | Kp (°C) | $^1$H-NMR ($CDCl_3$) δ [ppm] |
|---|---|---|---|---|---|---|---|---|---|
| 1 | $CH_3$ | H | H | $C_2H_5$ | H | A | 65 | 192 - 194 | 1,07 (m, 3 H); 2,20 (s, 1,71 H); 2,26 (s, 1,29 H); 3,42 (m, 2H); 5,26 (s, 1,18 H); 5,32 (s, 0,82 H); 5,97 (d, 0,48 H); 6,00 (s, 0,52 H); 7,30 (d, 0,41 H); 7,36 (d, 0,59 H) |
| 2 | $CH_3$ | $CH_3$ | H | $C_2H_5$ | H | A | 74 | 202 - 205 | 1,09 (m, 3 H); 1,92 (s, 3 H); 2,12 (s, 1,85 H); 2,16 (s, 1,15 H); 3,42 (m, 2H); 5,21 (s, 1,19 H); 5,32 (s, 0,81 H); 7,18 (s, 1 H) |
| 3 | $CH_3$ | H | H | $C_3H_7$ | H | B | 43 | 242 - 244 | 0,80 (m, 3 H); 1,46 (m, 2 H); 2,21 (s, 1,78 H); 2,27 (s, 1,22 H); 3,34 (m, 2H); 5,27 (s, 1,2 H); 5,34 (s, 0,8 H); 5,98 (d, 0,42 H); 6,02 (d, 0,58 H); 7,31 (d, 0,41 H); 7,36 (d, 0,59 H) |
| 4 | $CH_3$ | H | H | $C_4H_9$ | H | B | 38 | 232 - 233 | 0,82 (m, 3 H); 1,27 (m, 2 H); 1,48 (m, 2 H) 2,26 (s, 1,71 H); 2,32 (s, 1,29 H); 3,42 (m, 2H); 5,31 (s, 1,2 H); 5,38 (s, 0,8 H); 6,03 (d, 0,44 H); 6,06 (d, 0,56 H); 7,35 (d, 0,38 H); 7,36 (d, 0,63 H) |
| 5 | $CH_3$ | $CH_3$ | H | $C_8H_{17}$ | H | A | 81 | > 300 | 0,84 (m, 3 H); 1,20-1,45 (m, 12 H); 1,95 (s, 3H) 2,25 (s, 1,74 H); 2,32 (s, 1,26 H); 3,39 (m, 2 H); 5,23 (s, 1,2 H); 5,32 (s, 0,8 H); 7,21 (s, 1 H) |
| 6 | $CH_3$ | H | H | $C_6H_{13}$ | H | A | 78 | 251 - 253 | 0,85 (m, 3 H); 1,24 -1,51 (m, 8 H); 2,28 (s, 2,4 H); 2,34 (s, 0,6 H); 3,44 (m, 2 H); 5,40 (m, 2 H); 6,08 (m, 1 H); 7,43 (d, 1 H); |
| 7 | $CH_3$ | Cl | H | $C_4H_9$ | H | B | 52 | 252-254 | 0,84 (m, 3 H); 1,30 (m, 2 H); 1,51 (m, 2 H) 2,23 (s, 1,71 H); 2,33 (s, 1,29 H); 3,42 (m, 2 H); 5,21 (s, 1,1 H); 5,38 (s, 0,9 H); 7,36 (s, 0,4 H); 7,48 (s, 0,6 H); |
| 8 | $CH_3$ | Cl | H | $C_8H_{17}$ | H | A | 61 | > 300 | 0,85 (m, 3 H); 1,22 -1,50 (m, 12 H); 2,22 (s, 1,65 H); 2,29 (s, 1,35 H); 3,42 (m, 2 H); 5,26 (s, 1,1 H); 5,35 (s, 0,9 H); 7,35 (s, 0,4 H); 7,46 (s, 0,6 H) |

| Nr. | $R^1$ | $R^2$ | $R^3$ | $R^4$ | $R^5$ | Var. | Ausb. (%) | Kp (°C) | $^1H$-NMR ($CDCl_3$) δ [ppm] |
|---|---|---|---|---|---|---|---|---|---|
| 9 | $CH_3$ | H | H | Cyclo-hexyl | H | A | 77 | 262 - 265 | 1,17 -1,70 (m, 10 H); 2,28 (s, 1,65 H); 2,29 (s, 1,35 H); 3,38 (m, 1 H); 5,33 (s, 1,15 H); 5,40 (s, 0,85 H); 6,02 (d, 1 H); 7,35 (d, 1 H); |
| 10 | $CH_3$ | $CH_3$ | H | $C_4H_9$ | $CH_3$ | C | 63 | 229 -231 | 0,81 (t, 3 H); 1,24 - 1,63 (m, 7 H); 1,93 (d, 3 H); 2,18 (d, 3 H); 3,25 (m, 2 H); 5,29 (q, 0,87 H); 5,49 (q, 0,13 H); 7,21 (m, 1 H); |
| 11 | $CH_3$ | H | H | $C_2H_5$ | $CH_3$ | D | 71 | 158 -161 | 1,04 (m, 3 H); 1,55 (m, 3 H); 2,25 (m, 3 H); 3,25 (m, 2 H); 5,35 (q, 0,88 H); 5,49 (q, 0,12 H); 6,00 (d, 1 H); 7,38 (d, 1 H); |
| 12 | $CH_3$ | H | H | $C_2H_5$ | $CH_2Cl$ | D | 55 | 229 - 232 | 1,14 (m, 3 H); 2,25 (s, 1,79 H); 2,34 (s, 1,21 H); 3,25 (m, 2 H); 3,83 (m, 2 H); 5,35 (m, 1 H); 6,06 (d, 1 H); 7,44 (s, 1 H); |
| 13 | $CH_3$ | $CH_3$ | H | $C_2H_5$ | $CH_2Cl$ | D | 48 | 240 - 241 | 1,14 (m, 3 H); 1,96 (s, 3 H); 2,15 ( s, 2 H); 2,22 (s, 1 H); 3,40 (m, 2 H); 3,85 (m, 2 H); 5,28 (t, 0,61 H); 5,40 (t, 0,39 H); 7,25 (d, 1 H); |
| 14 | $CH_3$ | $CH_3$ | H | $CH_3$ | Ph | D | 62 | 254-258 | 1,94 (m, 4 H); 2,20 (s, 2 H); 3,40 (s, 3 H); 6,19 (s, 0,7 H) 6,40 (s 0,3 H); 7,06 - 7,40 (m, 6 H) |
| 15 | $CH_3$ | $CH_3$ | H | $C_2H_5$ | $CH_3$ | D | 51 | 162 - 165 | 1,06 (m, 3 H); 1,53 (m, 3 H); 1,93 (m, 3 H); 2,11 (s, 2,44 H); 2,21 (s, 0,56 H); 3,25 (m, 2 H); 5,29 (q, 0,84 H); 5,49 (q, 0,16 H); 7,20 (s, 1 H); |

Ermittlung der nitrifikationshemmenden Wirkung

Beispiel 1

100 g lehmiger Sandboden werden mit den erfindungsgemäßen Wirkstoffen innig vermischt. Gleichzeitig werden zu diesen 100 g Boden 10 mg Harnstoff-N zugegeben. Anschließend wird der Boden auf 50 Gew.-% seiner maximalen Wasserkapazität eingestellt. Die Konzentration der erfindungsgemäßen Mittel betrug jeweils 6 ppm.

Nach 14-tägiger Inkubation dieser Boden-Wirkstoffmischung wurden die resultierenden Nitrat- und Ammoniumgehalte bestimmt und die prozentuale Hemmung zur Variante ohne Wirkstoff ermittelt. Die Berechnung der Hemmwirkung in Prozent erfolgt nach:

$$\frac{a - b}{a - c} \times 100 = \% \ \% \text{ Hemmung}$$

a Nitrit- und Nitratgehalt der Kontrolle
b Nitrit- und Nitratgehalt der Probe mit Wirkstoff
c Nitrit- und Nitratgehalt des Bodens

Die Ergebnisse gehen aus Tabelle 2 hervor.

Tabelle 2:

| Nitrifikationshemmung in Prozent nach 14 Tagen Inkubation | |
|---|---|
| Verbindung Nr. | % Hemmung nach 14 Tagen |
| 1 | 100 |
| 2 | 100 |
| 3 | 99 |
| 4 | 100 |
| 5 | 100 |
| 6 | 100 |
| 7 | 99 |
| 8 | 100 |
| 9 | 99 |
| 10 | 98 |
| 11 | 98 |
| 12 | 92 |
| 13 | 96 |
| 14 | 100 |
| 15 | 94 |

Beispiel 2

Die Prüfung der erfindungsgemäßen Pyrazolderivate erfolgte in den in Tabelle 3 ausgewiesenen Konzentrationen. Dabei wurde die nitrifikations-hemmende Wirkung in der Weise ermittelt, dass 100 g lufttrockener Boden auf 50 % der maximalen Wasserkapazität eingestellt wurden. Hierzu wurden jeweils 10 mg Harnstoff-N und die ausgewiesene Wirkstoffmenge unter möglichst homogener Verteilung zugesetzt. Aus der Bestimmung des Umsatzes von Ammonium-N zu Nitratstickstoff im Vergleich zur Variante ohne Inhibitor wurde die prozentuale Hemmung zum Tag X ermittelt.

Neben der prozentualen Hemmung wurde der $t_{50}$-Wert als Maßzahl der Wirksamkeit rechnerisch ermittelt. Der $t_{50}$-Wert gibt an, in wieviel Tagen 50 % des zugesetzten Amid- bzw. Ammoniumstickstoffs in Nitrat umgewandelt werden.

Tabelle 3:

| Nitrifikationshemmende Wirkung ausgewählter Verbindungen | | |
|---|---|---|
| Nr. der Verbindung | Wirkstoffkonzentration ppm | $t_{50}$-Wert (Tage) |
| 1 | 0,5<br>1,0 | 20,6<br>40,2 |
| 2 | 1,0 | 14,8 |
| 3 | 1,0 | 31,2 |
| 4 | 0,5<br>1,0 | 32,5<br>50,0 |
| 5 | 1,0 | 44,6 |
| 6 | 0,5<br>1,0 | 25,65<br>51,39 |
| 7 | 0,5<br>1,0 | 18,96<br>30,63 |
| 8 | 0,5 | 28,09 |
| 9 | 1,0 | 16,25 |
| 10 | 1,0 | 13,9 |
| 11 | 1,0 | 20,6 |
| 14 | 0,5<br>1,0 | 23,5<br>33,8 |

## Patentansprüche

**1.** Verwendung von 1H-Pyrazol-Derivaten mit der allgemeinen Formel (I)

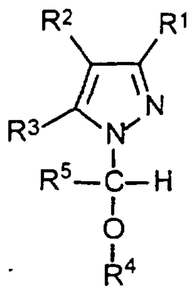

(I)

sowie deren Säureadditionsprodukten, wobei
$R^1$ und $R^3$ unabhängig voneinander für H oder einen $C_1$-$C_4$-Alkylrest,
$R^2$ für H, Halogen oder einen $C_1$-$C_4$-Alkylrest,
oder $R^1$ und $R^2$ zusammen für einen fünf- oder sechsgliedrigen Cycloalkylrest,
$R^4$ für $C_1$-$C_{20}$-Alkyl, $C_3$-$C_8$-Cycloalkyl, $C_6$-$C_{10}$-Aryl oder Alkylaryl mit $C_1$-$C_4$-Alkyl- und $C_6$-$C_{10}$-Arylgruppen und
$R^5$ für H, $C_1$-$C_{20}$-Alkyl, $C_3$-$C_8$-Cycloalkyl, $C_6$-$C_{10}$-Aryl oder Alkylaryl mit
$C_1$-$C_4$-Alkyl- und $C_6$-$C_{10}$-Arylgruppen stehen, wobei die Alkyl- und Arylreste von $R^4$ und $R^5$ durch Halogen, Hydroxyl, durch eine Trimethylsilyl-, Amino-, Nitro-, Cyano-, Carbonyl-, Carboxyl- oder eine Carboxyalkylgruppe mit 1 bis 5 C-Atomen substituiert sein können, zur Hemmung bzw. Steuerung der Nitrifikation.

**2.** Verwendung gemäß Anspruch 1, **dadurch gekennzeichnet, dass**
$R^1$ für einen $C_1$-$C_4$-Alkylrest,

$R^2$ für H, Halogen oder einen $C_1$-$C_4$-Alkylrest,
oder $R^1$ und $R^2$ zusammen für einen fünf- oder sechsgliedrigen Cycloalkylrest,
und $R^3$ für H stehen.

**3.** Verwendung nach Anspruch 1 oder 2, **dadurch gekennzeichnet, dass** $R^1$ und/oder $R^2$ einen Methylrest darstellen.

**4.** Verwendung nach den Ansprüchen 1 bis 3, **dadurch gekennzeichnet, dass** man als Säureadditionsprodukte der 1H-Pyrazol-Derivate die Umsetzungsprodukte mit Salzsäure, Phosphorsäure oder Schwefelsäure einsetzt.

**5.** Verwendung nach den Ansprüchen 1 bis 4, **dadurch gekennzeichnet, dass** die 1H-Pyrazol-Derivate oder ihre Säureadditionsprodukte zur Hemmung bzw. Steuerung der Nitrifikation in Kombination mit festen oder flüssigen ammonium- und/oder amidhaltigen Düngemitteln eingesetzt werden.

**6.** Zusammensetzung umfassend ein ammonium- und/oder amidhaltiges Düngemittel und ein 1-H-Pyrazol-Derivat der allgemeinen Formel (I) nach den Ansprüchen 1 bis 3 oder sein Säureadditionsprodukt.

**7.** Zusammensetzung nach Anspruch 6, **dadurch gekennzeichnet, dass** die 1H-Pyrazol-Derivate oder ihre Säureadditionsprodukte in einer Aufwandmenge von 0,1 bis 5,0 Gew.-%, insbesondere 0,5 bis 3,0 Gew.-%, berechnet auf den reduzierten Stickstoffgehalt des Düngemittels, eingesetzt werden.

**8.** Zusammensetzung nach den Ansprüchen 6 oder 7, **dadurch gekennzeichnet, dass** die 1H-Pyrazol-Derivate in fester oder flüssiger Form allein oder im Gemisch mit weiteren Zusatzstoffen in die Düngemittelschmelze oder -slurry während des Formgebungsprozesses eingebracht wurden und auf diese Weise homogen im Dünger verteilt vorliegen.

**9.** Zusammensetzung nach den Ansprüchen 6 oder 7, **dadurch gekennzeichnet, dass** die 1H-Pyrazol-Derivate oder ihre Säureadditionsprodukte als Lösung auf die Oberfläche der Düngemittelgranulate aufgebracht wurden und anschließend das Lösemittel durch Trocknung entfernt wurde.

**10.** Zusammensetzung nach den Ansprüchen 6 oder 7, **dadurch gekennzeichnet, dass** die 1H-Pyrazol-Derivate oder ihre Säureadditionsprodukte einem Flüssigdünger zugesetzt sind.

**11.** 1H-Pyrazol-Derivate der allgemeinen Formel (I)

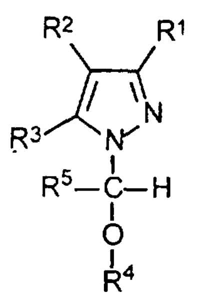

(I)

wobei $R^1$ für einen $C_1$-$C_4$-Alkylrest,
$R^2$ für H, Halogen oder einen $C_1$-$C_4$-Alkylrest,
oder $R^1$ und $R^2$ zusammen für einen fünf- oder sechsgliedrigen Cycloalkylrest,
$R^3$ für H,
$R^4$ für $C_1$-$C_{20}$-Alkyl, $C_3$-$C_8$-Cycloalkyl, $C_6$-$C_{10}$-Aryl oder Alkylaryl mit $C_1$-$C_4$-Alkyl- und $C_6$-$C_{10}$-Arylgruppen
und $R^5$ für H, $C_1$-$C_{20}$-Alkyl, $C_3$-$C_8$-Cycloalkyl, $C_6$-$C_{10}$-Aryl oder Alkylaryl mit $C_1$-$C_4$-Alkyl- und $C_6$-$C_{10}$-Arylgruppen stehen, wobei die Alkyl- und Arylreste von $R^4$ und $R^5$ durch Halogen, Hydroxyl, durch eine Trimethylsilyl-, Amino-, Nitro-, Cyano-, Carbonyl-, Carboxyl- oder eine Carboxyalkylgruppe mit 1 bis 5 C-Atomen substituiert sein können, sowie deren Säureadditionsprodukte.

**12.** Verfahren zur Herstellung der 1H-Pyrazol-Derivate nach Anspruch 11, **dadurch gekennzeichnet, dass** man die entsprechenden 1-unsubstituierten Pyrazole der Formel (II)

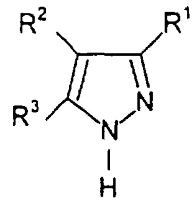

(II)

wobei $R^1$, $R^2$ und $R^3$ oben genannte Bedeutung besitzen, in einem organischen Lösemittel ausgewählt aus der Gruppe Ketone oder aromatische Kohlenwasserstoffe unter Zusatz von vorzugsweise molaren Mengen an Basen wie z. B. Alkalicarbonat mit einem α-Chlorether der allgemeinen Formel (III)

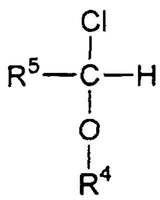

(III)

wobei $R^4$ und $R^5$ oben genannte Bedeutung besitzen, im Molverhältnis 1 : 1,0 - 1,2 bei 40 bis 100 °C umsetzt.

**13.** Verfahren zur Herstellung der 1H-Pyrazol-Derivate nach Anspruch 11, **dadurch gekennzeichnet, dass** man

a) die entsprechenden 1-unsubstituierten Basispyrazole der Formel (II)

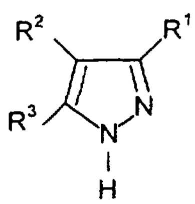

(II)

wobei $R^1$, $R^2$ und $R^3$ oben genannte Bedeutung besitzen, mit Aldehyden der allgemeinen Formel (IV)

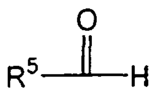

(IV)

wobei $R^5$ die oben genannte Bedeutung besitzt, gegebenenfalls in Gegenwart eines Lösemittels im Molverhältnis 1 : 1,0 - 1,2 bei Temperaturen von -30 bis 120 °C umsetzt und
b) die dabei entstehenden Pyrazol-Derivate der allgemeinen Formel (V),

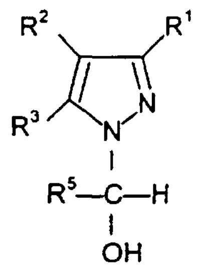

(V)

wobei $R^1$, $R^2$, $R^3$ und $R^5$ oben genannte Bedeutung besitzen, entweder direkt oder gegebenenfalls nach Isolierung mit Alkoholen $R^4OH$, wobei $R^4$ für $C_1$-$C_{20}$-Alkyl, $C_3$-$C_8$-Cycloalkyl steht, im Molverhältnis 1 : 1,0 bis 1,2 unter Zusatz von sauren Katalysatoren in aromatischen Kohlenwasserstoffen bei Temperaturen von 80 bis 150 °C reagieren lässt.

**14.** Verfahren nach Anspruch 13, **dadurch gekennzeichnet, dass** man bei der direkten Umsetzung in Stufe b) den Alkohol $R^4OH$ dem Reaktionsgemisch aus Stufe a) bei Temperaturen von -30 bis 10 °C zugibt.

**15.** Verfahren nach Anspruch 13, **dadurch gekennzeichnet, dass** als Katalysator p-Toluolsulfonsäure oder ein saurer Ionenaustauscher verwendet wird.

**16.** Verfahren zur Herstellung der 1H-Pyrazol-Derivate (mit $R^5 \neq H$) nach Anspruch 11, **dadurch gekennzeichnet, dass** man die 1-unsubstituierten Basispyrazole der Formel (II),

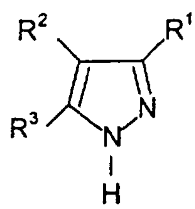

(II)

wobei $R^1$, $R^2$ und $R^3$ oben genannte Bedeutung besitzen, durch Erhitzen mit überschüssigem Aldehyddiacetal der Formel (VI),

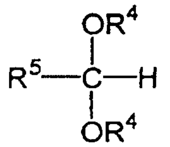

(VI)

wobei $R^4$ und $R^5$ oben genannte Bedeutung besitzen und $R^5 \neq H$ ist, im Molverhältnis 1 : 2 bis 5 unter Verwendung

von sauren Katalysatoren auf Temperaturen von 100 bis 130 °C zur Umsetzung bringt.

**17.** Verfahren nach Anspruch 16, **dadurch gekennzeichnet, dass** als Katalysator p-Toluolsulfonsäure verwendet wird.

**18.** Verfahren zur Herstellung der Pyrazol-Derivate nach Anspruch 11, **dadurch gekennzeichnet, dass** die Pyrazol-Derivate der allgemeinen Formel (V),

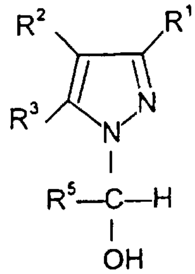

(V)

wobei $R^1$, $R^2$, $R^3$ und $R^5$ oben genannte Bedeutung besitzen, mit Alkyl- oder Cycloalkylhalogeniden $R^4$Hal, wobei $R^4$ für $C_1$-$C_{20}$-Alkyl, $C_3$-$C_8$-Cycloalkyl steht, im Molverhältnis 1 : 1 bis 1 : 3 unter Einsatz von vorzugsweise äquimolaren Mengen Alkalicarbonat bei Temperaturen von 50 bis 130 °C umgesetzt werden.

## Claims

**1.** Use of 1H-pyrazole derivatives having the general formula (I)

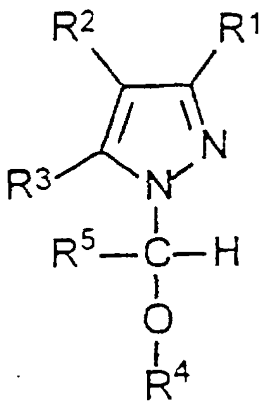

(I)

and the acid addition products thereof, in which $R^1$ and $R^3$, independently of one another, represent H or a $C_1$-$C_4$-alkyl radical,
$R^2$ represents H, halogen or a $C_1$-$C_4$-alkyl radical,
or $R^1$ and $R^2$ together represent a five- or six-membered cycloalkyl radical,
$R^4$ represents $C_1$-$C_{20}$-alkyl, $C_3$-$C_8$-cycloalkyl, $C_6$-$C_{10}$-aryl or alkylaryl having $C_1$-$C_4$-alkyl and $C_6$-$C_{10}$-aryl groups and
$R^5$ represents H, $C_1$-$C_{20}$-alkyl, $C_3$-Cg-cycloalkyl, $C_6$-$C_{10}$-aryl or alkylaryl having $C_1$-$C_4$-alkyl and $C_6$-$C_{10}$-aryl groups, it being possible for the alkyl and aryl radicals of $R^4$ and $R^5$ to be substituted by halogen, hydroxyl, by a trimethylsilyl, amino, nitro, cyano, carbonyl or carboxyl group or a carboxyalkyl group having 1 to 5 C atoms, for inhibiting or controlling nitrification.

**2.** Use according to Claim 1, **characterized in that**
$R^1$ represents a $C_1$-$C_4$-alkyl radical,
$R^2$ represents H, halogen or a $C_1$-$C_4$-alkyl radical,
or $R^1$ and $R^2$ together represent a five- or six-membered cycloalkyl radical,
and $R^3$ represents H.

**3.** Use according to Claim 1 or 2, **characterized in that** $R^1$ and/or $R^2$ represent a methyl radical.

**4.** Use according to any of Claims 1 to 3, **characterized in that** the reaction products with hydrochloric acid, phosphoric acid or sulphuric acid are used as acid addition products of the 1H-pyrazole derivatives.

**5.** Use according to any of Claims 1 to 4, **characterized in that** the 1H-pyrazole derivatives or their acid addition products are used for inhibiting or controlling nitrification in combination with solid or liquid ammonium- or amide-containing fertilizers.

**6.** Composition comprising an ammonium- and/or amide-containing fertilizer and a 1H-pyrazole derivative of the general formula (I) according to any of Claims 1 to 3 or its acid addition product.

**7.** Composition according to Claim 6, **characterized in that** the 1H-pyrazole derivatives or their acid addition products are used at an application rate of 0.1 to 5.0% by weight, in particular 0.5 to 3.0% by weight, based on the reduced nitrogen content of the fertilizer.

**8.** Composition according to either of Claims 6 and 7, **characterized in that** the 1H-pyrazole derivatives, in solid or liquid form, alone or as a mixture with further additives, were introduced into the fertilizer melt or slurry during the shaping process and are thus present homogeneously distributed in the fertilizer.

**9.** Composition according to either of Claims 6 and 7, **characterized in that** the 1H-pyrazole derivatives or their acid addition products were applied as a solution to the surface of the fertilizer granules and the solvent was then removed by drying.

**10.** Composition according to either of Claims 6 and 7, **characterized in that** the 1H-pyrazole derivatives or their acid addition products are added to a liquid fertilizer.

**11.** 1H-Pyrazole derivatives of the general formula (I)

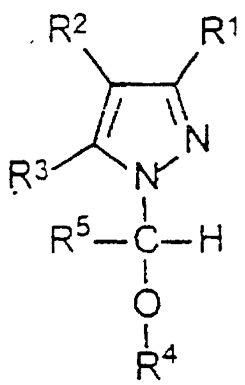
(I)

in which $R^1$ represents a $C_1$-$C_4$-alkyl radical,
$R^2$ represents H, halogen or a $C_1$-$C_4$-alkyl radical,
or $R^1$ and $R^2$ together represent a five- or six-membered cycloalkyl radical,
$R^3$ represents H,
$R^4$ represents $C_1$-$C_{20}$-alkyl, $C_3$-$C_8$-cycloalkyl, $C_6$-$C_{10}$-aryl or alkylaryl having $C_1$-$C_4$-alkyl and $C_6$-$C_{10}$-aryl groups and
$R^5$ represents H, $C_1$-$C_{20}$-alkyl, $C_3$-$C_8$-cycloalkyl, $C_6$-$C_{10}$-aryl or alkylaryl having $C_1$-$C_4$-alkyl and $C_6$-$C_{10}$-aryl groups, it being possible for the alkyl and aryl radicals of $R^4$ and $R^5$ to be substituted by halogen, hydroxyl, by a trimethylsilyl, amino, nitro, cyano, carbonyl or carboxyl group or a carboxyalkyl group having 1 to 5 C atoms, and

the acid addition products thereof.

**12.** Method for the production of the 1H-pyrazole derivatives according to Claim 11, **characterized in that** the corresponding 1-unsubstituted pyrazoles of the formula (II)

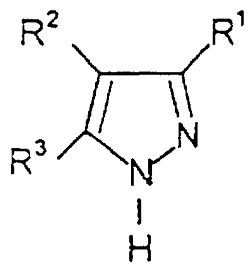

(II)

in which $R^1$, $R^2$ and $R^3$ have the above-mentioned meaning, are reacted, in an organic solvent selected from the group consisting of ketones or aromatic hydrocarbons, with addition of, preferably, molar amounts of bases, such as, for example, alkali metal carbonate, with an $\alpha$-chloroether of the general formula (III)

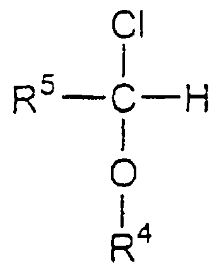

(III)

in which $R^4$ and $R^5$ have the above-mentioned meaning, in the molar ratio 1 : 1.0 - 1.2 at 40 to 100°C.

**13.** Method for the production of the 1H-pyrazole derivatives according to Claim 11, **characterized in that**

a) the corresponding 1-unsubstituted parent pyrazoles of the formula (II)

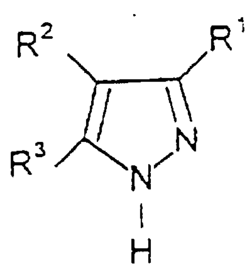

(II)

in which $R^1$, $R^2$ and $R^3$ have the above-mentioned meaning, are reacted with aldehydes of the general formula

(IV)

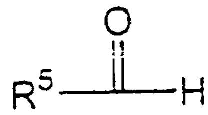

(IV)

in which $R^5$ has the above-mentioned meaning, optionally in the presence of a solvent in the molar ratio 1 : 1.0 - 1.2 at temperatures of -30 to 120°C and

b) the resulting pyrazole derivatives of the general formula (V)

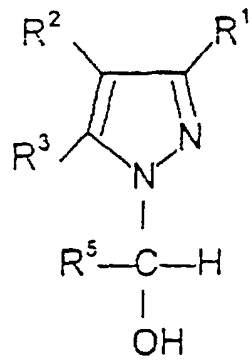

(V)

in which $R^1$, $R^2$, $R^3$ and $R^5$ have the above-mentioned meaning, are allowed to react, either directly or optionally after isolation, with alcohols of $R^4OH$, in which $R^4$ represents $C_1$-$C_{20}$-alkyl or $C_3$-$C_8$-cycloalkyl, in the molar ratio 1 : 1.0 to 1.2 with addition of acidic catalysts in aromatic hydrocarbons at temperatures of 80 to 150°C.

**14.** Method according to Claim 13, **characterized in that**, in the direct reaction in stage b), the alcohol $R^4OH$ is added to the reaction mixture from stage a) at temperature of -30 to 10°C.

**15.** Method according to Claim 13, **characterized in that** p-toluenesulphonic acid or an acidic ion exchanger is used as the catalyst.

**16.** Method for the production of the 1H-pyrazole derivatives (where $R^5 \neq H$) according to Claim 11, **characterized in that** the 1-unsubstituted parent pyrazoles of the formula (II)

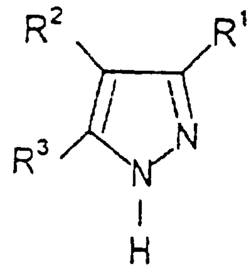

(II)

in which $R^1$, $R^2$ and $R^3$ have the above-mentioned meaning, are reacted by heating with excess aldehyde diacetal of the formula (VI)

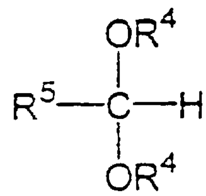

(VI)

in which $R^4$ and $R^5$ have the above-mentioned meaning and $R^5 \neq H$, in the molar ratio 1 : 2 to 5 using acidic catalysts at temperatures of 100 to 130°C.

**17.** Method according to Claim 16, **characterized in that** p-toluenesulphonic acid is used as the catalyst.

**18.** Method for the production of the pyrazole derivatives according to Claim 11, **characterized in that** the pyrazole derivatives of the general formula (V)

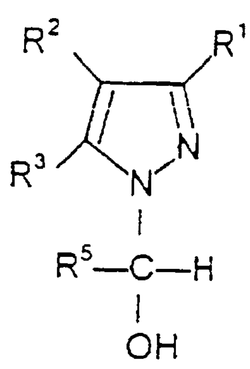

(V)

in which $R^1$, $R^2$, $R^3$ and $R^5$ have the above-mentioned meaning, are reacted with alkyl or cycloalkyl halides $R^4Hal$, in which $R^4$ represents $C_1$-$C_{20}$-alkyl or $C_3$-$C_8$-cycloalkyl, in the molar ratio 1 : 1 to 1 : 3 using, preferably, equimolar

amounts of alkali metal carbonate at temperatures of 50 to 130°C.

## Revendications

1. Utilisation de dérivés de pyrazole 1H de formule générale (I)

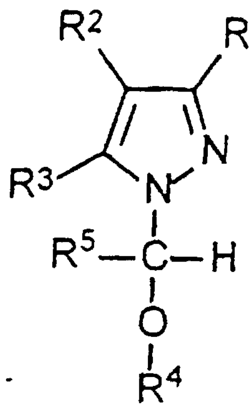

(I)

ainsi que de leurs produits d'addition d'acide, $R^1$ et $R^3$ représentant indépendamment l'un de l'autre H ou un radical alkyle $C_1$-$C_4$,
$R^2$ représentant H, l'halogène ou un radical alkyle $C_1$-$C_4$, ou $R^1$ et $R^2$ représentant ensemble un radical cycloalkyle à cinq ou six éléments,
$R^4$ représentant de l'alkyle $C_1$-$C_4$, du cycloalkyle $C_3$-$C_8$, de l'aryle $C_6$-$C_{10}$ ou de l'alkylaryle avec des groupes alkyle $C_1$-$C_4$ et aryle $C_6$-$C_{10}$ et $R^5$ représentant H, de l'alkyle $C_1$-$C_{20}$, du cycloalkyle $C_3$-$C_8$, de l'aryle $C_6$-$C_{10}$ ou de l'alkylaryle avec des groupes alkyle $C_1$-$C_4$ et aryle $C_6$-$C_{10}$, les radicaux alkyle et aryle de $R^4$ et $R^5$ pouvant être substitués par de l'halogène, de l'hydroxyle, par un groupe triméthylsilyle, amino, nitro, cyano, carbonyle, carboxyle ou carboxyalkyle ayant 1 à 5 atomes de C, afin de bloquer ou de contrôler la nitrification.

2. Utilisation selon la revendication 1, **caractérisée en ce que**
$R^1$ représente un radical alkyle $C_1$-$C_4$,
$R^2$ représente H, de l'halogène ou un radical alkyle $C_1$-$C_4$
ou $R^1$ et $R^2$ représentent ensemble un radical cycloalkyle à cinq ou six éléments,
et $R^3$ représente H.

3. Utilisation selon la revendication 1 ou 2,
**caractérisée en ce que**
$R^1$ et/ou $R^2$ représentent un radical méthyle.

4. Utilisation selon les revendications 1 à 3, **caractérisée en ce qu'**on met en oeuvre, comme produits d'addition d'acide des dérivés de pyrazole 1H, les produits de transposition avec de l'acide salique, de l'acide phosphorique ou de l'acide sulfurique.

5. Utilisation selon les revendications 1 à 4, **caractérisée en ce que** les dérivés de pyrazole 1H ou leurs produits d'addition d'acide sont mis en oeuvre pour bloquer ou contrôler la nitrification en combinaison avec des engrais solides ou liquides contenant de l'ammonium et/ou de l'amide.

6. Composition comprenant un engrais contenant de l'ammonium et/ou de l'amide et un dérivé de pyrazole 1H de la formule générale (I) selon les revendications 1 à 3 ou son produit d'addition d'acide.

7. Composition selon la revendication 6, **caractérisée en ce que** les dérivés de pyrazole 1H ou leurs produits d'addition d'acide sont mis en oeuvre en quantité opérationnelle de 0,1 à 5,0 % en poids, notamment 0,5 à 3,0 % en poids, calculée en fonction de la quantité d'azote réduite contenue dans l'engrais.

**8.** Composition selon les revendications 6 ou 7, **caractérisée en ce que** les dérivés de pyrazole 1H ont été introduits sous forme solide ou liquide seuls ou mélangés à d'autres additifs dans la coulée ou la boue d'engrais pendant le processus de façonnage et sont de ce fait répartis de manière homogène dans l'engrais.

**9.** Composition selon les revendications 6 ou 7, **caractérisée en ce que** les dérivés de pyrazole 1H ou leurs produits d'addition d'acide ont été introduits sous forme de solution sur la surface des granulés d'engrais et qu'ensuite le solvant a été éliminé par séchage.

**10.** Composition selon les revendications 6 ou 7, **caractérisée en ce que** les dérivés de pyrazole 1H ou leurs produits d'addition d'acide sont ajoutés à un engrais liquide.

**11.** Dérivés de pyrazole 1H de formule générale (I)

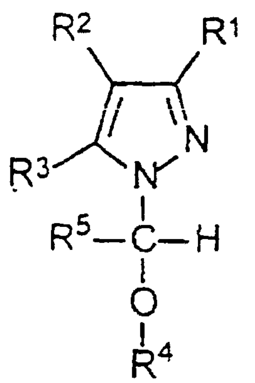

(I)

$R^1$ représentant un radical alkyle $C_1$-$C_4$,
$R^2$ représentant H, l'halogène ou un radical alkyle $C_1$-$C_4$, ou $R^1$ et $R^2$ représentant ensemble un radical cycloalkyle à cinq ou six éléments,
$R^3$ représentant H,
$R^4$ représentant de l'alkyle $C_1$-$C_4$, du cycloalkyle $C_3$-$C_8$, de l'aryle $C_6$-$C_{10}$ ou de l'akylaryle avec des groupes alkyle $C_1$-$C_4$ et aryle $C_6$-$C_{10}$ et $R^5$ représentant H, de l'alkyle $C_1$-$C_{20}$, du cycloalkyle $C_3$-$C_8$, de l'aryle $C_6$-$C_{10}$ ou de l'alkylaryle, avec des groupes alkyle $C_1$-$C_4$ et aryle $C_6$-$C_{10}$, les radicaux alkyle et aryle de $R^4$ et $R^5$ pouvant être substitués par de l'halogène, de l'hydroxyle, par un groupe triméthylsilyle, amino, nitro, cyano, carbonyle, carboxyle ou carboxyalkyle ayant 1 à 5 atomes de C, ainsi que leurs produits d'addition d'acide.

**12.** Procédé de préparation de dérivés de pyrazole 1H selon la revendication 11, **caractérisé en ce qu'**on transpose les pyrazoles non substitués 1 correspondants de la formule (II)

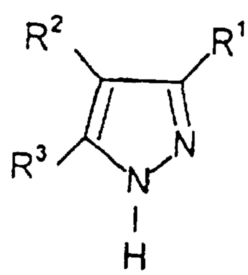

(II)

où $R^1$, $R^2$ et $R^3$ ont la signification indiquée plus haut, dans un solvant organique sélectionné parmi le groupe cétones ou hydrocarbures aromatiques, en ajoutant des quantités de préférence molaires de bases comme par exemple du carbonate alcalin, avec un éther de chlore α de formule générale (III)

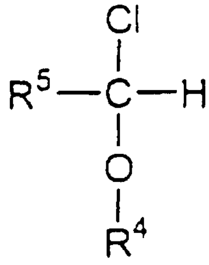

(III)

où $R^4$ et $R^5$ ont la signification indiquée plus haut, dans un rapport molaire de 1 :1,0 - 1,2, à 40 à 100° C.

**13.** Procédé de préparation de dérivés de pyrazole 1H selon la revendication 11, **caractérisé en ce qu'**on transpose

a) les pyrazoles de base non substitués 1 correspondants de la formule (II)

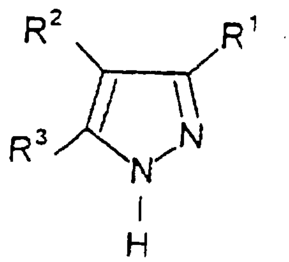

(II)

où $R^1$, $R^2$ et $R^3$ ont la signification indiquée plus haut, avec des aldéhydes de formule générale (IV)

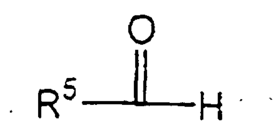

(IV)

où $R^5$ a la signification indiquée plus haut, éventuellement en présence d'un solvant, dans un rapport molaire de 1 : 1,0 - 1,2, à des températures de -30 à 120° C et
(b) qu'on fait réagir les dérivés de pyrazole 1H se formant alors, de formule générale (V)

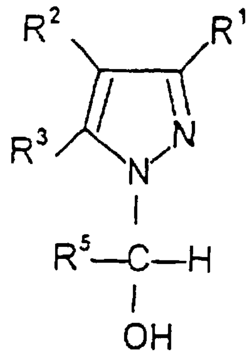

(V)

où $R^1$, $R^2$, $R^3$ et $R^5$ ont la signification indiquée plus haut, soit directement, soit éventuellement après isolation, avec des alcools $R^4OH$, $R^4$ représentant de l'alkyle $C_1$-$C_{20}$, du cycloalkyle $C_3$-$C_8$, dans un rapport molaire de 1 : 1,0 à 1,2, en ajoutant des catalyseurs acides dans des hydrocarbures aromatiques, à des températures de 80 à 150° C.

**14.** Procédé selon la revendication 13, **caractérisé en ce que**, lors de la transposition directe dans l'étape b), on ajoute l'alcool $R^4OH$ au mélange réactionnel de l'étape a) des températures de -30 à 10° C.

**15.** Procédé selon la revendication 13, **caractérisé en ce qu'**on utilise comme catalyseur de l'acide sulfonique de toluène p ou un échangeur ionique acide.

**16.** Procédé de préparation des dérivés de pyrazole 1H (avec $R^5$ = H) selon la revendication 11, **caractérisé en ce qu'**on transpose les pyrazoles de base non substitués 1 de la formule (II)

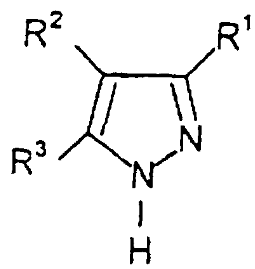

(II)

où $R^1$, $R^2$ et $R^3$ ont la signification indiquée plus haut, en chauffant avec de du diacétal d'aldéhyde excédentaire de formule (VI)

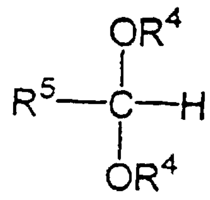

(VI)

où $R^4$ et $R^5$ ont la signification indiquée plus haut et où on a $R^5 \neq H$, dans un rapport molaire de 1 :2 à 5, en utilisant des catalyseurs acides, à des températures de 100 à 130° C.

**17.** Procédé selon la revendication 16, **caractérisé en ce qu'**on utilise comme catalyseur de l'acide sulfonique de toluène p.

**18.** Procédé de préparation des dérivés de pyrazole 1H selon la revendication 11, **caractérisé en ce qu'**on transpose les dérivés de pyrazole de formule générale (V)

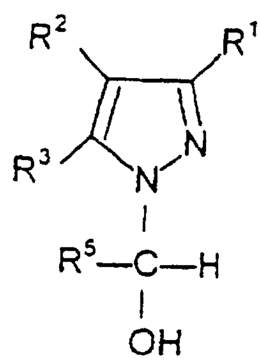

(V)

où $R^1$, $R^2$, $R^3$ et $R^5$ ont la signification indiquée plus haut, avec des halogénures d'alkyle ou de cycloalkyle $R^4Hal$, $R^4$ représentant de l'alkyle $C_1$-$C_{20}$, du cycloalkyle $C_3$-$C_8$, dans un rapport molaire de 1 : 1 à 1 : 3, en utilisant des quantités de préférence équimolaires de carbonate alcalin à des températures de 50 à 130° C.